# EUROPEAN PATENT SPECIFICATION

(11) **EP 2 334 283 B1**
(45) Date of publication and mention of the grant of the patent: **27.02.2013**
(21) Application number: 09741113.6
(22) Date of filing: 13.10.2009
(51) Int. Cl.: A61K 8/891, A61K 8/894, A61Q 17/04

(54) **RESILIENT PERSONAL CARE COMPOSITION COMPRISING POLYALKYL ETHER CONTAINING SILOXANE ELASTOMERS**
NACHGIEBIGE KÖRPERPFLEGEZUSAMMENSETZUNG MIT POLYALKYLETHER ENTHALTENDEN SILOXAN-ELASTOMEREN
COMPOSITION D' HYGIÈNE PERSONNELLE ELASTIQUE COMPRENANT DES ÉLASTOMÈRES DE SILOXANE DE TYPE POLYALKYLÉTHER

(30) Priority: 14.10.2008 US 251154
(43) Date of publication of application: 22.06.2011
(73) Proprietor: The Procter & Gamble Company, Cincinnati, OH 45202 (US)
(72) Inventor: BREYFOGLE, Laurie, Ellen, Milford, Ohio 45150 (US); TANNER, Paul, Robert, Lebanon, Ohio 45036 (US); AISTRUP, Elizabeth, Rebecca, Ohio 45213 (US)
(74) Representative: Engisch, Gautier
(86) International application number: PCT/US2009/060401
(87) International publication number: WO 2010/045163

(56) References cited:
- EP-A1- 1 426 027
- WO-A1-2006/110271
- WO-A1-2007/038993
- WO-A1-2007/132971
- WO-A1-2009/042732
- WO-A2-2007/109240
- WO-A2-2008/085360
- WO-A2-2009/006091
- WO-A2-2009/042832
- US-A- 5 412 004
- dow corning: "Dow Corning el-8051 in Silicone organic elastomer blend" 6 July 2009 (2009-07-06), XP002579646 Retrieved from the Internet: URL:www1.dowcorning.com/Datafiles/090007c8 802117f6.pdf> [retrieved on 2010-04-24]
- dow corning: "Dow corning el-8050 id silicone organic elastomer blend" 6 July 2009 (2009-07-06), XP002579647 Retrieved from the Internet: URL:www1.dowcorning.com/Datafiles/090007c8 802117d1.pdf> [retrieved on 2010-04-24]

## Description

### FIELD OF THE INVENTION

The present invention relates to a personal care composition in the form of an emulsion comprising a silicone elastomer with a polyalkyl ether crosslink (wherein the alkyl group has three or more carbon atoms in linear or branched orientation), a polar oil, and an aqueous phase.

### BACKGROUND OF THE INVENTION

An ongoing need exists to provide personal care compositions that prevent damage to the skin and other keratinous tissue from harmful ultraviolet radiation (UV). Much attention has been directed to improving the UV blocking efficacy of personal care compositions. Most of this work is focused on improving the efficacy and stability of the sunscreen actives within the compositions. While this work has resulted in significant improvements in protection, there are other factors that impact the UV blocking efficacy of personal care compositions. To provide adequate protection, these personal care compositions must be applied regularly and in a requisite amount. Therefore, a need exists for personal care compositions that encourage frequent use and ample use such as by having a pleasant feel without being oily or unstable.

The majority of highly effective organic sunscreens are oily or oil-soluble. These organic sunscreens are necessary for UV blocking, but have an unpleasant heavy, oily skin feel and present formulation difficulties such as compatibility with other components and product stability. To counter the heavy, oily skin feel associated with sunscreen compositions such products are commonly formulated as emulsions. The majority of such emulsions are oil-in-water emulsions wherein the aqueous phase (which in most cases is predominantly water) is thickened with polymeric thickeners. Considerably fewer sunscreen compositions in the form of an inverse emulsion (water-in-oil) exist.

WO 2007/038993 A1 (Schwan Stabilo Cosmetics GmbH) discloses a preparation in the form of a water-in-oil emulsion which contains: at least one silicone; as an emulsifier, at least one PEG/PPG dimethicone having from 15 to 20 units of PEG and PPG respectively; a lipid phase; from 0.1 to 5 wt.% water, based on the total weight of the composition; and, at least one particulate ingredient. In an embodiment, the preparation is used for light protection and comprises nano-pigments in combination with UV-A and / or UV-B filter substances.

US Patent No. 5,412,004 Tachibana Kiyomi et al.) describes: a silicone polymer which is prepared by addition polymerization; a paste-like silicone composition prepared by kneading the silicone polymer and silicone oil under a shearing force; and, a water-in-oil type cosmetic composition comprising the paste-like silicone composition as an oil phase component

WO 2006/110271 (Dow Corning) describes a water-in-silicone emulsion of which: i) the silicone phase contains an emollient, a silicone elastomer containing a hydrophilic group, and an optional nonionic surfactant; and, ii) the aqueous phase contains an anionic surfactant in an amount such that the ratio by weight of silicone elastomer to anionic surfactant in the emulsion is in the range from 60:1 to 1:1.

A further inverse emulsion, which is used as a rouge composition, is disclosed in WO 2007/132971 (Amorepacific Corp.)

Inverse emulsions present unique problems with viscosity and product stability. Thickening of the oil continuous phase with commercially available materials while providing consumer required skin feel is challenging. While it may be possible to thicken the oil phase using various types of waxy materials, the resulting emulsions are often instable at higher temperatures. Instability is further exacerbated by prolonged storage. Even if thickening and stability are achieved, most consumers do not prefer the look and feel of waxy personal care compositions. Consumers particularly dislike these personal care compositions if targeted for application to the face and neck.

To overcome these stability and aesthetic challenges water-in-oil emulsions may be thickened by including cross-linked silicone elastomers in the oil phase. Although they are relatively expensive, many cross-linked silicone elastomers are known to impart improved skin-feel to personal care compositions. Cross-linked silicone elastomers have the ability to swell and absorb a solvent thereby immobilizing the oil in the silicone phase and increasing the viscosity. The degree to which the cross-linked silicone elastomer swells is dependent on the chemical structure of the elastomer and solvent. When particles of the cross-linked silicone elastomer are chemically similar to the swelling solvent, the hydrodynamic volume of the elastomer increases due to absorption of more solvent; in the presence of a dissimilar solvent the elastomer particle shrinks which results in a lower viscosity oil phase and a less stable product.

Cross-linked silicone elastomers are typically categorized as non-emulsifying or emulsifying. Non-emulsifying cross-linked organopolysiloxane elastomers typically shrink in the presence of polar sunscreen oils making them a less desirable option for immobilization of oil. Typically, emulsifying cross-linked silicone elastomers, like substituted polyoxyethylene cross-linked elastomers, do not shrink as much in polar oils as do non-emulsifying cross-linked organopolysiloxane elastomers. However, emulsifying cross-linked silicone elastomers exhibit enough shrinkage to present formulation difficulties which impact desired consumer benefits. Any amount of shrinkage can destabilize the system which may impose limits on the amount of polar oil within the product. In cases where the polar oil is a sunscreen, the product may then have reduced or limited UV protection benefits. Furthermore, emulsifying cross-linked silicone elastomers are considerably more expensive and tend to have a more tacky or greasy skin feel. Given these limitations there is need for a structure agent with enhanced polar oil compatibility to stabilize water-in-oil emulsions that provide suitable skin feel benefits.

### SUMMARY OF THE INVENTION

In response of the problems identified in the Background, the present invention relates to a personal care composition in the form of a water-in-oil emulsion comprising polar oils and comprising polyalkyl ether cross-linked elastomers wherein the alkyl group is three or more units long. Personal care compositions with such elastomers exhibit increased stability and provide greater flexibility in formulation. This unexpected efficiency allows for the formation of water-in-oil emulsions with high levels of consumer beneficial components. In certain embodiments, the water-in-oil emulsions may comprise high levels of polar oil, high levels of water, or high levels of both. As will be seen in the Comparative Examples, the high levels of polar oil and/or aqueous phase were previously unachievable with conventional materials.

As defined in claim 1 appended hereto, the present invention relates to a stable personal care composition in the form of a water-in-oil emulsion comprising a) from 0.01% to 30% of a silicone elastomer comprising a polyalkyl ether group of Formula I or II: 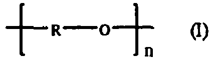 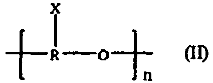 wherein R is an alkyl group having 3 or more carbon atoms in a linear or branched orientation and X is an amine, ester, amide, ketone, aldehyde, nitro, ether, enol, carboxyl, carbonyl, halide, benzyl, or aromatic substituent, and n is an integer between 2 and 30. The composition further comprises: b) from 0.1% to 20% of a non-emulsifying silicone elastomer; and, c) from 0.05% to 20% of an emulsifier. The composition further comprises: d) greater than 10% of a polar oil; a) and an aqueous phase, preferably greater than 10% aqueous phase.

### DETAILED DESCRIPTION OF THE INVENTION

The composition of the present invention may be used in skin care, cosmetic, and hair care products, non-limiting uses of which include moisturizers, conditioners, anti-aging compounds, skin lightening compounds, and combinations thereof. The composition is applied to keratinous tissue of the face, neck, hands, arms and other areas of the body exposed to ultraviolet radiation.

In all embodiments of the present invention, all percentages are by weight of the personal care composition, unless otherwise specified. All ratios are weight ratios, unless specifically stated otherwise. All numeric ranges are inclusive of narrower ranges. The number of significant digits conveys neither limitation on the indicated amounts nor on the accuracy of the measurements. All measurements are understood to be made at about 25 °C and at ambient conditions, where "ambient conditions" means conditions under about one atmosphere of pressure and at about 50% relative humidity.

"Personal care composition" means compositions suitable for topical application on mammalian keratinous tissue.

"Keratinous tissue," as used herein, refers to keratin-containing layers disposed as the outermost protective covering of mammals which includes, but is not limited to, skin, hair, nails, cuticles, etc.

"Stable" and "stability" refer to compositions which are substantially unaltered in chemical state, physical homogeneity and/or color, upon exposure to conditions reasonably expected to be incurred in shipping, storage and use, for example, for at least 30 days at a temperature of from about 0 °C to about 40 °C.

"Derivatives," as used herein, means ester, ether, amide and/or salt derivatives of the relevant compound.

"Polar," as used herein, means a material with a solubility parameter of greater than or equal to 7.4 (calories/cm³)^{0.5} to about 11 (calories/cm³)^{0.5}. "Non-polar," as used herein, means a material with a solubility parameter of less than 7.4 (calorieslcm³)^{0.5}. Solubility parameters are discussed in more detail by C. D. Vaughan in "The Solubility Parameter: What is it?," Cosmetics & Toiletries vol. 106, November, 1991, pp. 69-72. Solubility parameter may be determined or calculated according to procedures discussed within Barton, AFM (1991). Handbook of Solubility Parameters and Other Cohesion Parameters, 2nd edition. CRC Press.

### I. Personal Care Composition

The composition of the present invention is a water-in-oil emulsion. The oil phase of the present invention may comprise silicone oils, non-silicone oils such as hydrocarbon oils, esters, ethers, the like, and mixtures thereof. The personal care composition may have a viscosity of from about 10,000 cps (centipoise) to about 1,000,000 cps, alternatively from about 30,000 cps to about 500,000 cps, and alternatively from about 40,000 cps to about 200,000 cps.

### A. Silicone Elastomer Comprising a Polyalkyl Ether Group

The compositions of the present invention comprise a silicone elastomer comprising a polyalkyl ether pendant or a polyalkyl ether crosslink, wherein the alkyl group contains three or more carbon atoms. It has been found that use of such silicone elastomers provide multiple benefits over conventional silicone elastomers - either emulsifying or non-emulsifying - which are known in the art. The silicone elastomer comprising a polyalkyl ether pendant or a polyalkyl ether crosslink allow for improved flexibility in formulating. Compositions can be formed with high loading of aqueous phase materials and/or polar oil materials that have not been previously achieved with conventional silicone elastomers. Furthermore, these elastomers improve the skin-feel characteristics of the compositions.

The silicone elastomer comprises polysiloxane backbones connected by a crosslink. The polysiloxane backbone may be linear or branched. No specific restriction exists as to the type of polysiloxane composition that forms the backbone. The polysiloxane backbone may be an organopolysiloxane. Suitable organopolysiloxanes include methylvinylsiloxanes, methylvinylsiloxane-dimethylsiloxane copolymers, dimethylvinylsiloxy-terminated dimethylpolysiloxanes, dimethylvinylsiloxy-terminated dimethylsiloxane-methylphenylsiloxane copolymers, dimethylvinylsiloxy-terminated dimethylsiloxane-diphenylsiloxane-methylvinylsiloxane copolymers, trimethylsiloxy-terminated dimethylsiloxane-methylvinylsiloxane copolymers, trimethylsiloxy-terminated dimethylsiloxane-methylphenylsiloxane-methylvinylsiloxane copolymers, dimethylvinylsiloxy-terminated methyl(3,3,3-trifluoropropyl) polysiloxanes, and dimethylvinylsiloxy-terminated dimethylsiloxane-methyl(3,3,-trifluoropropyl) siloxane copolymers. The molecular weight of the polysiloxane backbone is not particularly limited.

In one embodiment, the polysiloxane backbone may comprise polyalkyl ether pendants wherein the alkyl group contains three or more carbon atoms. The polyalkyl ether pendants are connected to a polysiloxane backbone and do not interconnect polysiloxane backbones (i.e., crosslink). The polyalkyl ether may be generally represented by Formula I or Formula II: 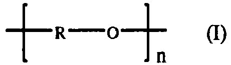 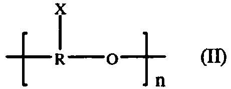 wherein R is an alkyl group having three or more carbon atoms in a linear or branched orientation; X is an amine, ester, amide, ketone, aldehyde, nitro, ether, enol, carboxyl, carbonyl, halide, benzyl, or aromatic substituent; and n is an integer between 2 and 30. In a suitable embodiment, R is either isopropyl (CH₃-CH-CH₂) or n-propyl (CH₂-CH₂-CH₂). In certain embodiments, the lower limit of n is greater than 5, 10, 15, or, alternately, 20. In certain embodiments, the polyalkyl ether may be joined directly to the polysiloxane backbone or indirectly through one or more other functional moieties. Exemplary silicone elastomers comprising a polyalkyl ether pendant may be synthesized according to the methods described in U.S. Pat. Nos. 5,837,793 and 5,811,487.

In another embodiment, the silicone elastomer comprises one or more crosslink between the polysiloxane backbones. In a preferred embodiment, these cross-links may comprise a polyalkyl ether wherein the alkyl group contains three or more carbon atoms. The polyalkyl ether crosslink may be generally represented by Formula I or Formula II, as presented above, wherein R is an alkyl group having three or more carbon atoms in a linear or branched orientation; X is an amine, ester, amide, ketone, aldehyde, nitro, ether, enol, carboxyl, carbonyl, halide, benzyl, or aromatic substituent; and n is an integer between 2 and 30. In a suitable embodiment, R is either isopropyl (CH₃-CH-CH₂) or n-propyl (CH₂-CH₂-CH₂). In certain embodiments, the lower limit of n is greater than 5, 10, 15, or, alternately, 20. The silicone elastomer may comprise additional crosslinking units other than polyalkyl ether (e.g., conventional divinyl crosslinking compounds). For example, the polyalkyl ether may be joined directly to the polysiloxane backbone or indirectly through one or more other crosslinking units. Furthermore, the alkyl ether units may represent the entirety of the crosslinking chain (i.e., a polyalkyl ether homopolymer crosslink). The alkyl ether units may be periodically or randomly distributed with other crosslinking monomers.

The personal care composition comprises at least about 0.01% of the silicone elastomer comprising polyalkyl ether groups wherein the alkyl group contains three or more carbon atoms. The maximum percentage of the silicone elastomer comprising polyalkyl ether groups may be practically limited by material costs and space consideration for other components. However, in accordance with the invention, the personal care composition comprises from about 0.01% to about 30% of the silicone elastomer comprising polyalkyl ether groups wherein the alkyl group contains three or more carbon atoms. In certain embodiments, the personal care composition may comprise from about 0.1% to about 20% or from about 0.5% to about 10% of the silicone elastomer comprising a polyalkyl ether groups wherein the alkyl group contains three or more carbon atoms. It should be recognized that the silicone elastomer comprising polyalkyl ether groups wherein the alkyl group contains three or more carbon atoms may be supplied pre-swollen with a solvent. With a pre-swollen swollen elastomer, the weight percentages recited above are of the elastomer alone (*i.e*., excluding the weight of the solvent).

Particularly suitable silicone elastomers comprising a polyalkyl ether group wherein the alkyl group contains three or more carbon atoms include compounds with the International Nomenclature of Cosmetic Ingredients (INCI) name: bis-vinyldimethicone/bis-isobutyl PPG-20 crosspolymer, bis-vinyldimethicone/PPG-20 crosspolymer, dimethicone/bis-isobutyl PPG-20 crosspolymer, dimethicone/PPG-20 crosspolymer, and dimethicone/bis-secbutyl PPG-20 crosspolymer. Such cross-linked elastomers are available from Dow Corning, Midland, MI, under the experimental names of SOEB-1, SOEB-2, SOEB-3 and SOEB-4, and under the proposed commercial name of DC EL-8052 IH Si Organic Elastomer Blend. The elastomer particles were supplied pre-swollen in the respective solvents, isododecane (for SOEB 1-2), isohexadecane (for SOEB-3), and isodecyl neopentanoate (for SOEB-4).

### B. Non-emulsifying silicone elastomer

The personal care composition of the present invention comprises a non-emulsifying silicone elastomer. More particularly, the personal care composition contains from about 0.1% to about 20%, alternatively from about 0.5% to about 15% or from about 1% to 10% of the non-emulsifying crosslinked organopolysiloxane elastomer. "Non-emulsifying silicone elastomer" means that the silicone elastomer comprises no polyoxyalkylene groups.

In an embodiment the non-emulsifying silicone elastomer comprises dimethicone crosspolymers, dimethicone/vinyl dimethicone crosspolymers, C30-45 alkyl cetearyl dimethicone crosspolymers, cetearyl dimethicone crosspolymers, dimethicone/phenyl vinyl dimethicone crosspolymers, vinyl dimethicone/lauryl dimethicone crosspolymers, trifluoropropyl dimethicone/trifluoropropyl divinyldimethicone crosspolymers, or mixtures thereof.

Suitable non-emulsifying silicone elastomers include the CTFA (Cosmetic, Toiletry, and Fragrance Association International Cosmetic Ingredient Dictionary and Handbook, 11 ed.) designated dimethicone/vinyl dimethicone crosspolymers such as supplied by Dow Corning™ (DC 9506), General Electric™ (SFE 839), Shin Etsu™ (KSG 15 and 16), and Grant Industries (GRANSIL™ RPS-NA) and dimethicone/phenyl vinyl dimethicone crosspolymer such as KSG 18 available from Shin Etsu™. Other exemplary silicone elastomers include the CTFA designated dimethicone crosspolymers including Dow Corning™ (DC 9040, DC 9041, DC 9045).

### C. Emulsifier

The personal care composition of the present invention comprises an emulsifier that may be linear, branched, and/or cross-linked. The personal care composition may comprise from about 0.05% to about 20% or from about 0.1% to about 10% total emulsifier. Emulsifiers may be nonionic, anionic or cationic. Non-limiting examples of emulsifiers are disclosed in U.S. Patent 3,755,560, U.S.

Patent 4,421,769, and McCutcheon's Detergents and Emulsifiers, North American Edition and International Edition, pages 235-246 (1993). Suitable emulsions may have a wide range of viscosities, depending on the desired product form.

Emulsifiers may also include emulsifying silicone elastomers other than the abovementioned silicone elastomer comprising a polyalkyl ether pendant or a polyalkyl ether crosslink, wherein the alkyl group contains three or more carbon atoms. Suitable emulsifying silicone elastomers include cross-linked organopolysiloxane elastomers having at least one polyalkyl ether or polyglycerolated unit. These cross-linked elastomers may also be co-modified to include alkyl substituents. Particularly useful emulsifying polyoxyethylene cross-linked elastomers include Shin Etsu's KSG-21, KSG-210, KSG-24, KSG-240, KSG-31, KSG-310, KSG-32, KSG-320, KSG-33, KSG-330, KSG-34, and KSG-340. Particularly useful emulsifying polyglycerolated cross-linked elastomers include Shin Etsu's KSG-710, KSG-810, KSG-820, KSG-830, and KSG-840. Other silicone emulsifying elastomers are supplied by Dow Corning™, including PEG-12 dimethicone crosspolymers (DC 9010 and 9011), lauryl PEG/PPG methicone (DC5200), and PEG-PPG-18 dimethicone (DC5225C).

Linear or branched type silicone emulsifiers are also useful in this application.

In an embodiment the emulsifier is of a linear or branched structure comprising polyether modified silicones, polyglycerin modified silicones, and polyether/alkyl co-modified silicones, or polyglycerin/alkyl co-modified silicones.

Particularly useful polyether modified elastomers include Shin Etsu's KF-6011, KF-6012, KF-6013, KF- 6015, KF-6015, KF-6017, KF-6043, KF-6028, and KF-6038. Also particularly useful are the polyglycerolated linear or branched siloxane emulsifiers including Shin Etsu's KF-6100, KF- 6104, KF-6105.

### D. Polar Oil

The composition of the present invention comprises a polar oil. In certain embodiments, the composition may comprise greater than about 10% of polar oil. In other embodiments, the composition may comprise greater than about 20% of polar oil. In certain embodiments, the polar oil may have a solubility parameter of from about 8.0 (calories/cm³)^{0.5} to about 10.5 (calories/cm³)^{0.5}.

Suitable polar oils include ethers, esters, amides, propoxylates, and mixtures thereof. The aforementioned oils may be saturated, unsaturated, aliphatic (straight or branched chains), alicyclic, or aromatic.

Suitable polar oils include but are not limited to, butyl and isopropyl phthalimide (Pelemol™ BIP), phenylethyl benzoate (X-tend™ 226), dicaprylyl carbonate (Tegosoft™ DEC), isopropyl lauroylsarcosinate (Eldew™ SL 205), butyl octylsalicylate (Hallbrite™ BHB), dioctyl malate, dicaprylyl maleate (Hallbrite™ DCM), di-isopropyl adipate, isononyl isononanoate, isopropyl isostearate, propylene glycol dicaprate, C12-15 alcohol benzoate (Finsolv TN), PPG-11 stearyl ether, and derivatives and mixtures thereof.

Sunscreens are another example of polar oils. Exemplary sunscreens include but are not limited to, benzophenone-3, bis-ethylhexyloxyphenol methoxyphenyl triazine, butyl methoxydibenzoyl-methane, diethylamino hydroxy-benzoyl hexyl benzoate, ethylhexyl triazone, drometrizole trisiloxane, ethylhexyl methoxy-cinnamate, ethylhexyl salicylate, octocrylene, homosalate, polysilicone-15, 2-ethylhexyl N,N-dimethyl-p-aminobenzoate, 4-isopropyl dibenzoylmethane, 3-(4-methylbenzylidene) camphor, 3-benzylidene camphor, and menthyl anthranilate.

In one embodiment, the polar oil comprises (i) a sunscreen, the sunscreen comprising benzophenone-3, bis-ethylhexyloxyphenol methoxyphenyl triazine, butyl methoxydibenzoyl-methane, diethylamino hydroxy-benzoyl hexyl benzoate, ethylhexyl methoxy-cinnamate, ethylhexyl salicylate, ethylhexyl triazone, octocrylene, homosalate, polysilicone-15, or mixtures thereof; and (ii) a member comprising butyl phthalimide, isopropyl phthalimide, phenylethyl benzoate, dicaprylyl carbonate, isopropyl lauroylsarcosinate, butyl octylsalicylate, dioctyl malate, dicaprylyl maleate, di-isopropyl adipate, isononyl isononanoate, isopropyl isostearate, propylene glycol dicaprate, C12- 15 alcohol benzoate, derivatives or mixtures thereof.

In one embodiment, the polar oil comprises at least one oil-soluble sunscreen which, in its commercially-available purified form, is an oil-soluble crystalline and/or solid compound. It is to be understood that the oil-soluble crystalline and/or solid sunscreen is substantially dissolved, and thus does not remain in a crystalline form, in the personal care composition of the present invention. For example, a suitable polar oil may comprise a combination of an oil-soluble crystalline sunscreen and a solvent such as butyl methoxydibenzoyl-methane with isopropyl lauroylsarcosinate. Particularly suitable crystalline sunscreens include benzophenone-3, bis- ethylhexyloxyphenol methoxyphenyl triazine, butyl methoxydibenzoyl-methane, diethylamino hydroxy-benzoyl hexyl benzoate, ethylhexyl methoxy-cinnamate, ethylhexyl salicylate, ethylhexyl triazone, octocrylene, homosalate, and polysilicone-15.

Other exemplary polar oils include retinoid such as retinol and retinol propionate, tocopherol (i.e., vitamin E), derivatives of tocopherol, and tetrahexyldecyl ascorbate.

### E. Aqueous Phase

The personal care composition of the present invention contains an aqueous phase. The personal care composition may comprise at least about 10% aqueous phase. A unique aspect of the personal care composition is that greater loading of the aqueous phase can be obtained while still providing ample formulation space for other ingredients such as polar oils and silicones. In certain embodiments, the personal care composition may comprise from about 10% to about 50% aqueous phase. Alternately, the personal care composition may comprise from about 15% to about 40% aqueous phase, and most preferably from about 20% to about 35% aqueous phase. Within the emulsion the aqueous phase is the internal or discontinuous phase.

The aqueous phase typically comprises water. The aqueous phase may be comprised entirely of water. In other embodiments, the aqueous phase may comprise components other than water (i.e. non-water components), including but not limited to water-soluble moisturizing agents, conditioning agents, anti-microbials, humectants and/or other water-soluble skin care actives, to impart an increased benefit to the keratinous tissue. In one embodiment, the aqueous phase of the personal care composition comprises a humectant such as glycerin and/or other polyols.

### F. Waxes

The personal care composition of the present invention may further comprise a wax. Waxes suitable for use herein include but are not limited to animal, vegetable, mineral, synthetic, or silicone waxes. In certain embodiments, a hydrophobic wax may be preferred. Suitable hydrophobic waxes include but are not limited to long-chain hydrocarbons and substituted hydrocarbons, including those with ester, alcohol, acid, ketone, amide, amine, ether, carboxyl, halogen, or aldehyde functional groups. When used, the personal care composition may comprise from about 0.1% to about 40%, or, alternatively, from about 0.5% to about 10%, of a wax.

### G. Optional Ingredients

### 1. Particulate Material

The compositions of the present invention may comprise from about 0.001% to about 40%, alternatively from about 3% to about 30%, and alternatively from about 5% to about 20%, of one or more particulate materials. Non-limiting examples of suitable powders include inorganic powders (for example, iron oxides, titanium dioxides, zinc oxides, silica), organic powders, composite powders, optical brightener particles, and mixtures of any of the foregoing. These particulates can, for instance, be platelet shaped, spherical, elongated or needle-shaped, or irregularly shaped; surface coated or uncoated; porous or non-porous; charged or uncharged; and can be added to the current compositions as a powder or as a pre-dispersion. In one embodiment, the particulate material is hydrophobically coated.

Suitable organic powders particulate materials include, but are not limited, to polymeric particles chosen from the methylsilsesquioxane resin microspheres, for example, Tospearl™ 145A, (Toshiba Silicone); microspheres of polymethylmethacrylates, for example, Micropearl™ M 100 (Seppic); the spherical particles of crosslinked polydimethylsiloxanes, for example, Trefil™ E 506C or Trefil™ E 505C (Dow Corning Toray Silicone); spherical particles of polyamide, for example, nylon-12, and Orgasol™ 2002D Nat C05 (Atochem); polystyrene microspheres, for example Dyno Particles, sold under the name Dynospheres™, and ethylene acrylate copolymer, sold under the name FloBead™ EA209 (Kobo); aluminum starch octenylsuccinate, for example Dry Flo™ (National Starch); microspheres of polyethylene, for example Microthene™ FN510-00 (Equistar), silicone resin, polymethylsilsesquioxane silicone polymer, platelet shaped powder made from L-lauroyl lysine, and mixtures thereof.

The composition of the present invention further may comprise interference pigments, including hydrophobically-modified interference pigments. Herein, "interference pigments" means thin, plate-like layered particles having two or more layers of controlled thickness. The layers have different refractive indices that yield a characteristic reflected color from the interference of typically two, but occasionally more, light reflections, from different layers of the plate-like particle. One example of interference pigments are micas layered with about 50 - 300 nm films of TiO₂, Fe₂O₃, silica, tin oxide, and/or Cr₂O₃ and include pearlescent pigments. Interference pigments are available commercially from a wide variety of suppliers, for example, Rona (Timiron^{™} and Dichrona^{™}), Presperse (Flonac^{™}), Englehard (Duochrome^{™}), Kobo (SK-45-R and SK-45-G), BASF (Sicopearls^{™}) and Eckart (Prestige^{™}). In one embodiment, the average diameter of the longest side of the individual particles of interference pigments is less than about 75 microns, and alternatively less than about 50 microns.

Non-limiting examples of suitable colorants include iron oxides, ferric ammonium ferrocyanide, manganese violet, ultramarine blue, and chromium oxide, phthalocyanine blue and green pigment, encapsulated dyes, inorganic white pigments, for example TiO₂, ZnO, or ZrO₂, FD&C dyes, D&C dyes, and mixtures thereof.

### 2. Inorganic Sunscreens

The composition further may comprise from about 0.001% to about 10%, and alternatively from about 0.1% to about 5%, of an inorganic and/or oil-insoluble sunscreen. Non-limiting examples of suitable insoluble sunscreens include methylene bis-benzotriazolyl tetramethylbutyl-phenol, titanium dioxides, zinc cerium oxides, zinc oxides, and derivatives and mixtures thereof.

### 3. Skin Care Actives

Suitable skin care actives include, but are not limited to, vitamins, peptides, sugar amines, sunscreens, oil control agents, tanning actives, anti-acne actives, desquamation actives, anti-cellulite actives, chelating agents, skin lightening agents, flavonoids, protease inhibitors, non-vitamin antioxidants and radical scavengers, hair growth regulators, anti-wrinkle actives, anti-atrophy actives, minerals, phytosterols and/or plant hormones, tyrosinase inhibitors, anti-inflammatory agents, N-acyl amino acid compounds, antimicrobials, and antifungals. These skin care actives are provided in further detail in U.S. application publication No. US2006/0275237A1 and US2004/ 0175347A1.

Particularly suitable skin actives include vitamin B3 compounds, sugar amines, peptides, and hexamidine. As used herein, "vitamin B₃ compound" means a compound having the formula: 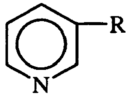 wherein R is - CONH₂ (*i.e*., niacinamide), - COOH (*i.e*., nicotinic acid) or - CH₂OH (*i.e.,* nicotinyl alcohol); derivatives thereof; and salts of any of the foregoing. As used herein, "sugar amine" includes isomers and tautomers of such and its salts (*e.g*., HCl salt) and its derivatives. Examples of sugar amines include glucosamine, N-acetyl glucosamine, mannosamine, N-acetyl mannosamine, galactosamine, N-acetyl galactosamine, their isomers (*e.g*., stereoisomers), and their salts (*e.g*., HCl salt). As used herein, "peptide" refers to peptides containing ten or fewer amino acids and their derivatives, isomers, and complexes with other species such as metal ions (*e.g*., copper, zinc, manganese, magnesium, and the like). The compositions of the present invention can include hexamidine compounds, its salts, and derivatives. As used herein, "hexaminide compound" means a compound having the formula: 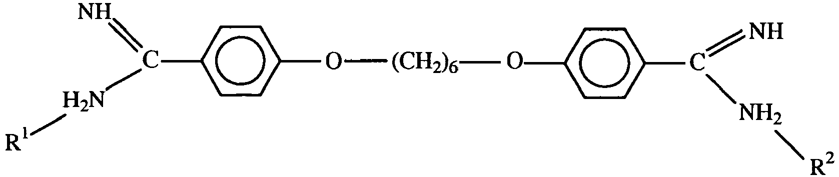 wherein R¹ and R² are optional or are organic acids (*e.g*., sulfonic acids, etc.).

### 4. Non-polar Emollient

The composition of the present invention comprises from about 10% to about 70%, and alternatively 30% to about 50% of a non-polar emollient. Non-limiting examples of suitable non-polar emollients include silicone oils, hydrocarbon oils, and mixtures thereof. Useful non-polar emollients in the present invention include natural, synthetic, saturated, unsaturated, straight chained, branched chained, linear, cyclic, aromatic, volatile, and non-volatile non-polar emollients, and mixtures thereof.

Non-limiting examples of suitable non-polar hydrocarbons oils include mineral oils and branched chain hydrocarbons (such as commercially available, for example, under the tradenames Permethyl™ (Permethyl Corporation™) and Isopar™ (Exxon™)).

Non-limiting examples of suitable non-polar silicone oils include linear and cyclic dimethicones. Commercially available examples of these types of silicones include the Dow Corning 200 series, Dow Corning 344, and Dow Corning 345 (all available from Dow Corning™ Corp.); and SF1202, SF1204, and the Viscasil™ series (all available from the G.E. Silicones™). Additional non-polar silicone oils include alkyl (for example, 2 carbons to 30 carbons) and aryl (for example, phenyl or styrenyl) substituted silicones, including by not limited to phenyl methicone, phenyl dimethicone, phenyl trimethicone, diphenyl dimethicone, phenylethyl dimethicone, hexyl dimethicone, lauryl dimethicone, cetyl dimethicone, stearyl dimethicone, bis-stearyl dimethicone, and mixtures thereof.

### II. Methods

The present invention therefore also further relates to methods of protecting human skin from the harmful effects of UV radiation. Such methods generally involve attenuating or reducing the amount of UV radiation which reaches the skin's surface. The personal care compositions of the present invention are suitable for use as a sunscreen to provide protection to human skin from the harmful effects of UV radiation which may include, but are not limited to, sunburn and premature aging of the skin.

The personal care compositions may be used to improve or regulate the condition of keratinous tissue. Conditions to be improved or regulated include increasing the luminosity or "glow" of the skin, reducing the appearance of wrinkles and coarse deep lines, fine lines, crevices, bumps, and large pores; thickening of keratinous tissue (e.g., building the epidermis and/or dermis and/or sub-dermal layers of the skin, and where applicable the keratinous layers of the nail and hair shaft, to reduce skin, hair, or nail atrophy); increasing the convolution of the dermal-epidermal border (also known as the rete ridges); preventing loss of skin or hair elasticity, for example, due to loss, damage and/or inactivation of functional skin elastin, resulting in such conditions as elastosis, sagging, loss of skin or hair recoil from deformation; reduction in cellulite; change in coloration to the skin, hair, or nails, for example, under-eye circles, blotchiness (e.g., uneven red coloration due to, for example, rosacea), sallowness, discoloration caused by telangiectasia or spider vessels, dryness, brittleness, and graying hair.

To protect the skin from UV radiation, a safe and effective (photoprotective) amount of the composition is topically applied to the skin. "Topical application" refers to application of the present compositions by spreading, spraying, etc. onto the surface of the skin. The exact amount applied may vary depending on the level of UV protection desired. According to one suitable method, from about 0.5 mg of the personal care composition per cm² of skin to about 25 mg of personal care composition per cm² of skin are typically applied. According to one suitable method, 2 mg of the personal care composition per cm² of skin is recommended to achieve appropriate SPF rating. Application should be repeated based on the user's sensitivity to the sun or susceptibility to burning.

### Examples

The following describe non-limiting examples of the personal care composition. The reported percentages indicate the weight of the component expressed as a percentage of the total weight of the personal care composition. Each Example may comprise one or more of the optional ingredients in amounts also disclosed herein. The Examples may be prepared as follows: In a suitable vessel combine the aqueous-phase ingredients and heat to 50 °C with gentle mixing. In a separate vessel, combine the sunscreens (octisalate, homosalate, octocrylene, avobenzone) and sunscreen solvent (isopropyl lauroylsarcosinate) and heat to 50 °C with gentle mixing. When the solutions in both vessels are translucent and free of particulates to the naked eye; allow them to cool to room temperature. Then, using the sunscreen premix created in the previous step, combine all of the ingredients for the silicone phase in a suitable container and mix until smooth and homogeneous. Add the premade water phase to the smooth silicone phase and mix until homogeneous. Vigorous mixing may be required. Finally add powders as needed to the emulsion and mix until smooth and homogeneous. Pour product into suitable containers and store at room temperature.

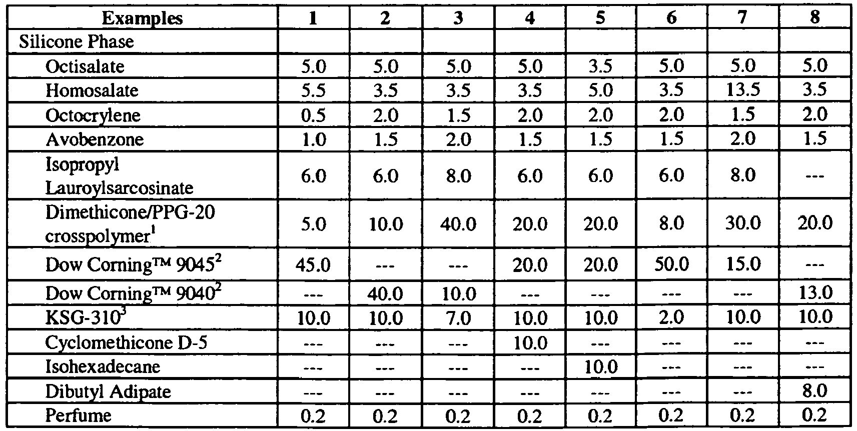

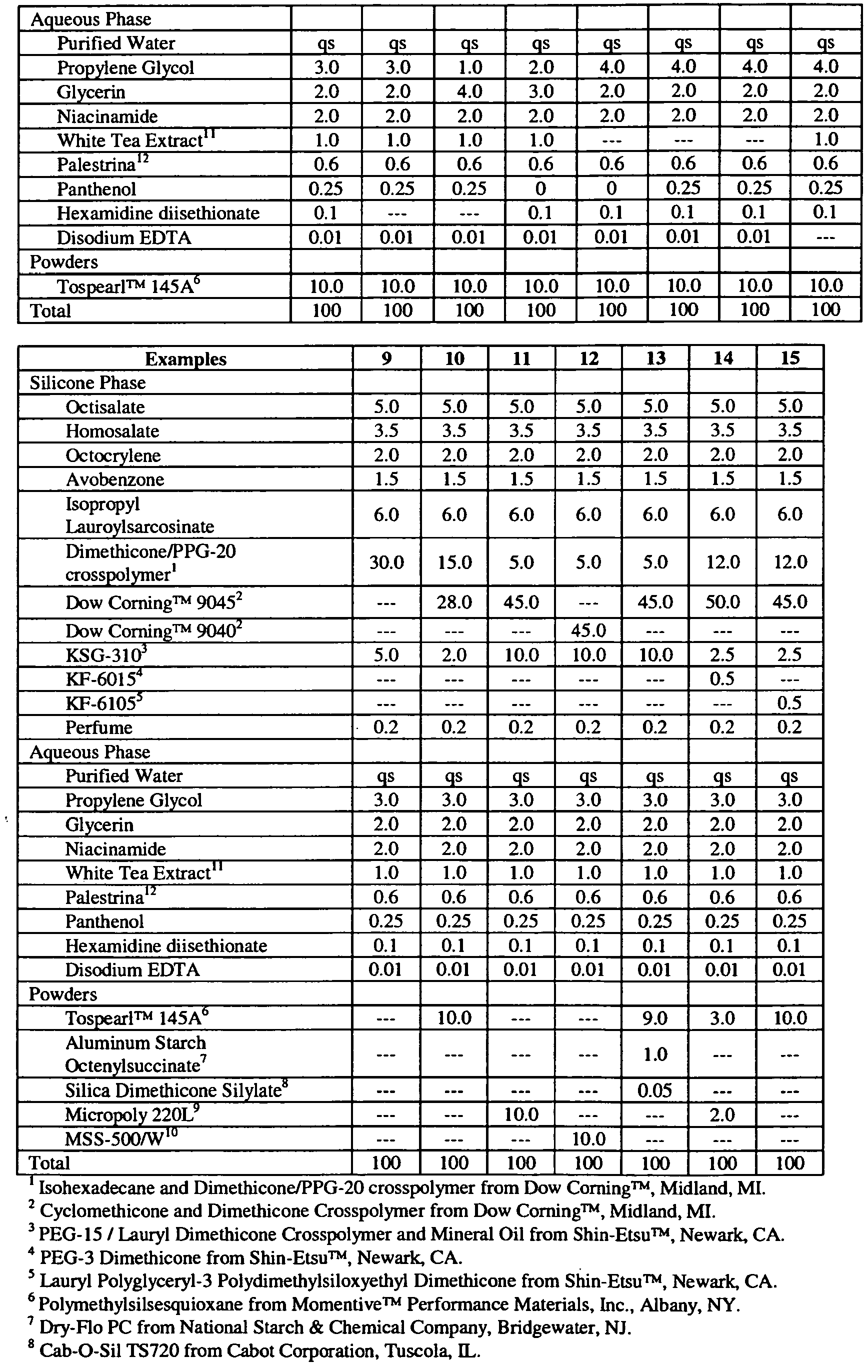

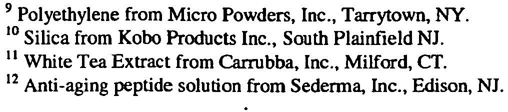

### Comparative Examples

The following is a comparison between examples falling within the present invention and comparative examples using conventional materials. The reported percentages indicate the weight of the component expressed as a percentage of the total weight of the personal care composition. The Comparative Examples may comprise one or more of the optional ingredients in amounts also disclosed herein. The Comparative Examples may be prepared by the methods used in preparation of the Examples above from the following components. Product stability is determined via visual assessment. A batch the size of 400 g is prepared and placed in a round container with the dimensions of 3.0" tall and 4.5" in diameter. A divot in the shape of a three-dimensional rectangle that is 1" wide, by 4.5" long, and 1" deep is cut into the product. The sample remains at room temperature and is visually monitored to determine if liquid synereses from the bulk product into the divot over time. If no syneresis is observed after 8 or more days, product stability is deemed 'Stable'; if syneresis occurs after 2 days but on or before 7 days, product stability is deemed 'Marginal'; and if syneresis occurs within 48 hours or less, product stability is deemed 'Unacceptable'. For perspective, in cases where product stability is 'unacceptable' the quantity of oil syneresed may be large enough that it can be poured or pipetted from the divot.

Comparative Examples 1-4 highlight the stability advantage gained when a small amount of a polypropyl ether cross-linked siloxane elastomer is included within formulation (E1, falling within the present invention). The same stability advantage is not attained with equivalent levels of other non-emulsifying or emulsifying elastomers including a dimethicone crosspolymer (DC9045)(C2), polyalkyl ether cross-linked elastomer (KSG-210)(C3), or a polyglycerol cross-linked elastomer (KSG-710) (C4). C2-C4 fall outside the present invention.

| **Comparative Examples** | | **E1** | **C2** | **C3** | **C4** |
|---|---|---|---|---|---|
| Silicone Phase | | | | | |
| | Octisalate | 5.0 | 5.0 | 5.0 | 5.0 |
| | Homosalate | 3.5 | 3.5 | 3.5 | 3.5 |
| | Octocrylene | 2.0 | 2.0 | 2.0 | 2.0 |
| | Avobenzone | 1.5 | 1.5 | 1.5 | 1.5 |
| | Isopropyl Lauroylsarcosinate | 6.0 | 6.0 | 6.0 | 6.0 |
| | Dimethicone/PPG-20 crosspolymer¹ | 10.0 | --- | --- | --- |
| | Dow Corning™ 9045² | --- | 10.0 | --- | --- |
| | Dow Corning™ 9040² | 40.0 | 40.0 | 40.0 | 40.0 |
| | KSG-310³ | 10.0 | 10.0 | 10.0 | 10.0 |
| | KSG-210⁴ | --- | --- | 10.0 | --- |
| | KSG-710⁵ | --- | --- | --- | 10.0 |
| | Perfume | 0.2 | 0.2 | 0.2 | 0.2 |
| Aqueous Phase | | | | | |
| | Purified Water | qs | qs | qs | qs |
| | Propylene Glycol | 3.0 | 3.0 | 3.0 | 3.0 |
| | Glycerin | 2.0 | 2.0 | 2.0 | 2.0 |
| | Niacinamide | 2.0 | 2.0 | 2.0 | 2.0 |
| | White Tea Extract⁶ | 1.0 | 1.0 | 1.0 | 1.0 |
| | Palestrina⁷ | 0.6 | 0.6 | 0.6 | 0.6 |
| | Panthenol | 0.25 | 0.25 | 0.25 | 0.25 |
| | Hexamidine diisethionate | 0.1 | 0.1 | 0.1 | 0.1 |
| | Disodium EDTA | 0.01 | 0.01 | 0.01 | 0.01 |

| Powders | | | | | |
|---|---|---|---|---|---|
| | Tospearl™ 145A⁸ | 10.0 | 10.0 | 10.0 | 10.0 |

| Total | | 100 | 100 | 100 | 100 |
|---|---|---|---|---|---|
| **Product Stability** | | Stable | Unacceptable | Unacceptable | Unacceptable |

| | | | | | |
|---|---|---|---|---|---|
| ¹ Isohexadecane and Dimethicone/PPG-20 crosspolymer from Dow Corning™, Midland, MI. ² Cyclomethicone and Dimethicone Crosspolymer from Dow Corning™, Midland, MI. ³ PEG-15 / Lauryl Dimethicone Crosspolymer and Mineral Oil from Shin-Etsu™, Newark, CA. ⁴ Dimethicone and Dimethicone/PPG-10/15 Crosspolymer from Shin-Etsu™, Newark, CA. ⁵ Dimethicone and Dimethicone/Polyglycerin-3 Crosspolymer from Shin-Etsu™, Newark, CA. ⁶ White Tea Extract from Carrubba, Inc., Milford, CT. ⁷ Anti-aging peptide solution from Sederma, Inc., Edison, NJ.. ⁸ Polymethylsilsesquioxane from Momentive™ Performance Materials, Inc., Albany, NY. | | | | | |

Comparative Examples 5-7 demonstrate the formula flexibility gained when a small amount of a polypropyl ether crosslinked siloxane elastomer is included within the formulation (E5 - falling within the present invention). This flexibility is demonstrated by accommodating higher levels of aqueous phase while maintaining good product stability. The same advantage is not attained with equivalent levels of polyalkyl ether cross-linked siloxane elastomer (KSG-210)(C6), or a polyglycerol cross-linked siloxane elastomer (KSG-710)(C7). C6-C7 fall outside the present invention.

| **Comparative Examples** | | **E5** | **C6** | **C7** |
|---|---|---|---|---|
| Silicone Phase | | | | |
| | Octisalate | 5.0 | 5.0 | 5.0 |
| | Homosalate | 3.5 | 3.5 | 3.5 |
| | Octocrylene | 2.0 | 2.0 | 2.0 |
| | Avobenzone | 1.5 | 1.5 | 1.5 |
| | Isopropyl Lauroylsarcosinate | 6.0 | 6.0 | 6.0 |
| | Dimethicone/PPG-20 crosspolymer¹ | 20.0 | --- | --- |
| | Dow Corning™ 9045² | --- | --- | --- |
| | Dow Corning™ 9040² | 9.5 | 9.5 | 9.5 |
| | KSG-310³ | 10.0 | 10.0 | 10.0 |
| | KSG-210⁴ | --- | 20.0 | --- |
| | KSG-710⁵ | --- | --- | 20.0 |
| | KF-6105⁶ | 0.5 | 0.5 | 0.5 |
| | Perfume | 0.2 | 0.2 | 0.2 |

| Aqueous Phase | | | | |
|---|---|---|---|---|
| | Purified Water | qs | qs | qs |
| | Propylene Glycol | 3.0 | 3.0 | 3.0 |
| | Glycerin | 2.0 | 2.0 | 2.0 |
| | Niacinamide | 2.0 | 2.0 | 2.0 |
| | White Tea Extract⁷ | 1.0 | 1.0 | 1.0 |
| | Palestrina⁸ | 0.6 | 0.6 | 0.6 |
| | Panthenol | 0.25 | 0.25 | 0.25 |
| | Hexamidine diisethionate | 0.1 | 0.1 | 0.1 |
| | Disodium EDTA | 0.01 | 0.01 | 0.01 |

| Powders | | | | |
|---|---|---|---|---|
| | Tospearl™ 145A⁹ | 10.0 | 10.0 | 10.0 |
| Total | | 100 | 100 | 100 |
| **Product Stability** | | Stable | Unacceptable | Unacceptable |

| | | | | |
|---|---|---|---|---|
| ¹ Isohexadecane and Dimethicone/PPG-20 crosspolymer from Dow Corning™, Midland, MI. ² Cyclomethicone and Dimethicone Crosspolymer from Dow Corning™, Midland, MI. ³ PEG-15 / Lauryl Dimethicone Crosspolymer and Mineral Oil from Shin-Etsu™, Newark, CA. ⁴Dimethicone and Dimethicone/PPG-10/15 Crosspolymer from Shin-Etsu™, Newark, CA. ⁵ Dimethicone and Dimethicone/Polyglycerin-3 Crosspolymer from Shin-Etsu™, Newark, CA. ⁶ Lauryl Polyglyceryl-3 Polydimethylsiloxyethyl Dimethicone from Shin-Etsu™, Newark, CA. ⁷ White Tea Extract from Carrubba, Inc., Milford, CT. ⁸ Anti-aging peptide solution from Sederma, Inc., Edison, NJ. ⁹ Polymethylsilsesquioxane from Momentive™ Performance Materials, Inc., Albany, NY. | | | | |

Comparative Examples 8-10 demonstrate the formula flexibility gained when a small amount of a polypropyl ether crosslinked siloxane elastomer is included within the formulation (E8 - falling within the present invention). This flexibility is demonstrated through accommodation of higher levels of sunscreen actives and solvents while maintaining good product stability. The same advantage is not attained with equivalent levels of polyalkyl ether cross-linked siloxane elastomer (KSG-210)(C9), or a polyglycerol cross-linked siloxane elastomer (KSG-710)(C10). C9-C10 fall outside the present invention.

| **Comparative Examples** | | **E8** | **C9** | **C10** |
|---|---|---|---|---|
| Silicone Phase | | | | |
| | Octisalate | 7.1 | 7.1 | 7.1 |
| | Homosalate | 4.9 | 4.9 | 4.9 |
| | Octocrylene | 2.9 | 2.9 | 2.9 |
| | Avobenzone | 2.1 | 2.1 | 2.1 |
| | Isopropyl Lauroylsarcosinate | 6.0 | 6.0 | 6.0 |
| | Dimethicone/PPG-20 crosspolymer¹ | 20.0 | --- | --- |
| | Dow Corning™ 9045² | --- | --- | --- |
| | Dow Corning™ 9040² | 25.0 | 25.0 | 25.0 |
| | KSG-310³ | 10.0 | 10.0 | 10.0 |
| | KSG-210⁴ | --- | 20.0 | --- |
| | KSG-710⁵ | --- | --- | 20.0 |

| Aqueous Phase | | | | |
|---|---|---|---|---|
| | Purified Water | qs | qs | qs |
| | Perfume | 0.2 | 0.2 | 0.2 |
| | Propylene Glycol | 13.0 | 13.0 | 13.0 |
| | Glycerin | 2.0 | 2.0 | 2.0 |
| | Niacinamide | 2.0 | 2.0 | 2.0 |
| | White Tea Extract⁶ | 1.0 | 1.0 | 1.0 |
| | Palestrina⁷ | 0.6 | 0.6 | 0.6 |
| | Panthenol | 0.25 | 0.25 | 0.25 |
| | Hexamidine diisethionate | 0.1 | 0.1 | 0.1 |
| | Disodium EDTA | 0.01 | 0.01 | 0.01 |

| Powders | | | | |
|---|---|---|---|---|
| | Tospearl™ 145A⁸ | 10.0 | 10.0 | 10.0 |
| Total | | 100 | 100 | 100 |
| **Product Stability** | | Stable | Unacceptable | Unacceptable |

| | | | | |
|---|---|---|---|---|
| ¹Isohexadecane and Dimethicone/PPG-20 crosspolymer from Dow Corning™, Midland, MI. ² Cyclomethicone and Dimethicone Crosspolymer from Dow Corning™, Midland, MI. ³ PEG-15 / Lauryl Dimethicone Crosspolymer and Mineral Oil from Shin-Etsu™, Newark, CA. ⁴Dimethicone and Dimethicone/PPG-10/15 Crosspolymer from Shin-Etsu™, Newark, CA. ⁵ Dimethicone and Dimethicone/Polyglycerin-3 Crosspolymer from Shin-Etsu™, Newark, CA. ⁶ White Tea Extract from Carrubba, Inc., Milford, CT. ⁷ Anti-aging peptide solution from Sederma, Inc., Edison, NJ. ⁸ Polymetuylsilsesquioxane from Momentive™ Performance Materials, Inc., Albany, NY. | | | | |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

To the extent that any meaning or definition of a term in this written document conflicts with any meaning or definition of the term in a document referred herein as background art, the meaning or definition assigned to the term in this written document shall govern.

Whereas particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A stable personal care composition in the form of a water-in-oil emulsion comprising:
a) from 0.01wt% to 30wt% of a silicone elastomer comprising a polyalkyl ether group of Formula I or II: 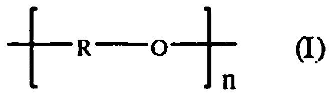 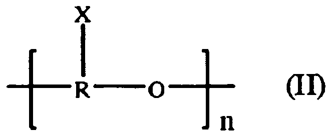 wherein R is an alkyl group having 3 or more carbon atoms in a linear or branched orientation, preferably wherein R is isopropyl or n-propyl and wherein X is an amine, ester, amide, ketone, aldehyde, nitro, ether, enol, carboxyl, carbonyl, halide, benzyl, or aromatic substituent, and wherein n is an integer between 2 and 30;
b) from 0.1wt% to 20wt% of a non-emulsifying silicone elastomer;
c) from 0.05wt% to 20wt% of an emulsifier;
d) greater than 10wt% of a polar oil; and
e) an aqueous phase comprising water.

2. The stable personal care composition of Claim 1, wherein the silicone elastomer comprising the polyalkyl ether group comprises a polysiloxane backbone and a pendant chain extending from the polysiloxane, wherein the pendant chain comprises the polyalkyl ether group.

3. The stable personal care composition of Claim 1, wherein the silicone elastomer comprising the polyalkyl ether group comprises at least two polysiloxane backbones connected by a cross-link chain, wherein the cross-link chain comprises the polyalkyl ether group.

4. The stable personal care composition of Claim 1, wherein the silicone elastomer comprising the polyalkyl ether group is selected from a group consisting of bis-vinyldimethicone/bis- isobutyl PPG-20 crosspolymer, bis-vinyldimethicone/PPG-20 crosspolymer, dimethicone/bis-isobutyl PPG-20 crosspolymer, dimethicone/PPG-20 crosspolymer, and dimethicone/bis-secbutyl PPG-20 crosspolymer.

5. The stable personal care composition according to any one of the preceding claims, wherein the non-emulsifying silicone elastomer comprises dimethicone crosspolymers, dimethicone/vinyl dimethicone crosspolymers, C30-45 alkyl cetearyl dimethicone crosspolymers, cetearyl dimethicone crosspolymers, dimethicone/phenyl vinyl dimethicone crosspolymers, vinyl dimethicone/lauryl dimethicone crosspolymers, trifluoropropyl dimethicone/trifluoropropyl divinyldimethicone crosspolymers, or mixtures thereof.

6. The stable personal care composition according to any one of the preceding claims, wherein the emulsifier is a cross-linked silicone emulsifier without a polyalkyl ether group of Formula I or II, wherein R is an alkyl group having 3 or more carbon atoms in a linear or branched orientation and X is an amine, ester, amide, ketone, aldehyde, nitro, ether, enol, carboxyl, carbonyl, halide, benzyl, or aromatic substituent, and n is an integer between 2 and 30; preferably said emulsifier is a polyoxyalkylenated or polyglycerolated crosslinked silicone elastomer.

7. The stable personal care composition according to any of Claims 1 to 6, wherein the emulsifier is of a linear or branched structure comprising polyether modified silicones, polyglycerin modified silicones, and polyether/alkyl co-modified silicones, or polyglycerin/alkyl co-modified silicones.

8. The stable personal care composition according to any one of the preceding claims, wherein the polar oil comprises a sunscreen, preferably the sunscreen comprises benzophenone-3, bis-ethylhexyloxyphenol methoxyphenyl triazine, butyl methoxydibenzoyl-methane, diethylamino hydroxy-benzoyl hexyl benzoate, ethylhexyl methoxy-cinnamate, ethylhexyl salicylate, ethylhexyl triazone, octocrylene, homosalate, polysilicone-15, or mixtures thereof.

9. The stable personal care composition according to any of Claims 1 to 7, wherein the polar oil comprises butyl phthalimide, isopropyl phthalimide, phenylethyl benzoate, dicaprylyl carbonate, isopropyl lauroylsarcosinate, butyl octylsalicylate, dioctyl malate, dicaprylyl maleate, di-isopropyl adipate, isononyl isononanoate, isopropyl isostearate, propylene glycol dicaprate, C12- 15 alcohol benzoate, derivatives or mixtures thereof.

10. The stable personal care composition according to any of Claims 1 to 7, wherein the polar oil comprises:
(i) a sunscreen, the sunscreen comprising benzophenone-3, bis-ethylhexyloxyphenol methoxyphenyl triazine, butyl methoxydibenzoyl-methane, diethylamino hydroxy-benzoyl hexyl benzoate, ethylhexyl methoxy-cinnamate, ethylhexyl salicylate, ethylhexyl triazone, octocrylene, homosalate, polysilicone-15, or mixtures thereof; and
(ii) a member comprising butyl phthalimide, isopropyl phthalimide, phenylethyl benzoate, dicaprylyl carbonate, isopropyl lauroylsarcosinate, butyl octylsalicylate, dioctyl malate, dicaprylyl maleate, di-isopropyl adipate, isononyl isononanoate, isopropyl isostearate, propylene glycol dicaprate, C12-15 alcohol benzoate, derivatives or mixtures thereof.

11. The stable personal care composition according to any one of the preceding claims, further comprising a skin care active, the skin care active comprising vitamins, peptides, sugar amines, oil control agents, tanning actives, anti-acne actives, desquamation actives, anti-cellulite actives, chelating agents, skin lightening agents, flavonoids, protease inhibitors, non-vitamin antioxidants and radical scavengers, hair growth regulators, anti-wrinkle actives, anti-atrophy actives, minerals, phytosterols and/or plant hormones, tyrosinase inhibitors, anti-inflammatory agents, N-acyl amino acid compounds, antimicrobials, antifungals, or mixtures thereof.

12. The stable personal care composition according to any one of the preceding claims, wherein the composition comprises from 10wt% to 50wt% aqueous phase.

## Patentansprüche

1. Stabile Körperpflegezusammensetzung in der Form einer Wasser-in-Öl-Emulsion, umfassend:
a) von 0,01 Gew.-% bis 30 Gew.-% ein Silikonelastomer, umfassend eine Polyalkylethergruppe der Formel I oder II: 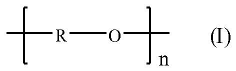 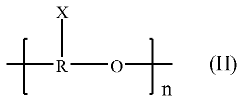 wobei R eine Alkylgruppe mit 3 oder mehr Kohlenstoffatomen in einer linearen oder verzweigten Ausrichtung ist, wobei vorzugsweise R Isopropyl oder n-Propyl ist und wobei X ein Amin-, Ester-, Amid-, Keton-, Aldehyd-, Nitro-, Ether-, Enol-, Carboxyl-, Carbonyl-, Halogenid-, Benzyl- oder aromatischer Substituent ist und wobei n eine ganze Zahl zwischen 2 und 30 ist;
b) von 0,1 Gew.-% bis 20 Gew.-% ein nichtemulgierendes Silikonelastomer;
c) von 0,05 Gew.-% bis 20 Gew.-% einen Emulgator;
d) zu mehr als 10 Gew.-% ein polares Öl; und
e) eine Wasserphase, die Wasser umfasst.

2. Stabile Körperpflegezusammensetzung nach Anspruch 1, wobei das Silikonelastomer, das die Polyalkylethergruppe umfasst, eine Polysiloxan-Hauptkette und eine seitenständige Kette, die von dem Polysiloxan ausgeht, umfasst, wobei die seitenständige Kette die Polyalkylethergruppe umfasst.

3. Stabile Körperpflegezusammensetzung nach Anspruch 1, wobei das Silikonelastomer, das die Polyalkylethergruppe umfasst, mindestens zwei Polysiloxan-Hauptketten umfasst, die durch eine Vernetzungskette miteinander verbunden sind, wobei die Vernetzungskette die Polyalkylethergruppe umfasst.

4. Stabile Körperpflegezusammensetzung nach Anspruch 1, wobei das Silikonelastomer, das die Polyalkylethergruppe umfasst, ausgewählt ist aus einer Gruppe bestehend aus Bisvinyldimethicon/Bisisobutyl-PPG-20-Kreuzpolymer, Bisvinyldimethicon/PPG-20-Kreuzpolymer, Dimethicon/Bisisobutyl-PPG-20-Kreuzpolymer, Dimethicon/PPG-20-Kreuzpolymer und Dimethicon/Bissecbutyl-PPG-20-Kreuzpolymer.

5. Stabile Körperpflegezusammensetzung nach einem der vorstehenden Ansprüche, wobei das nichtemulgierende Silikonelastomer Dimethicon-Kreuzpolymere, Dimethicon/Vinyldimethicon-Kreuzpolymere, C30-45-Alkylcetearyldimethicon-Kreuzpolymere, Cetearyldimethicon-Kreuzpolymere, Dimethicon/Phenylvinyldimethicon-Kreuzpolymere, Vinyldimethicon/Lauryldimethicon-Kreuzpolymere, Trifluorpropyldimethicon/Trifluorpropyldivinyldimethicon-Kreuzpolymere oder Mischungen davon umfasst.

6. Stabile Körperpflegezusammensetzung nach einem der vorstehenden Ansprüche, wobei der Emulgator ein vernetzter Silikonemulgator ohne Polyalkylethergruppe der Formel I oder II ist, wobei R eine Alkylgruppe mit 3 oder mehr Kohlenstoffatomen in einer linearen oder verzweigten Ausrichtung ist und X ein Amin-, Ester-, Amid-, Keton-, Aldehyd-, Nitro-, Ether-, Enol-, Carboxyl-, Carbonyl-, Halogenid-, Benzyl- oder aromatischer Substituent ist und n eine ganze Zahl zwischen 2 und 30 ist; wobei der Emulgator vorzugsweise ein polyoxyalkyleniertes oder polyglyceroliertes vernetztes Silikonelastomer ist.

7. Stabile Körperpflegezusammensetzung nach einem der Ansprüche 1 bis 6, wobei der Emulgator eine lineare oder verzweigte Struktur aufweist, die polyethermodifizierte Silikone, polyglycerinmodifizierte Silikone und polyether/alkyl-comodifizierte Silikone oder polyglycerin/alkyl-comodifizierte Silikone umfasst.

8. Stabile Körperpflegezusammensetzung nach einem der vorstehenden Ansprüche, wobei das polare Öl ein Sonnenschutzmittel umfasst, wobei das Sonnenschutzmittel vorzugsweise Benzophenon-3, Bisethylhexyloxyphenolmethoxyphenyltriazin, Butylmethoxydibenzoylmethan, Diethylaminohydroxybenzoylhexylbenzoat, Ethylhexylmethoxycinnamat, Ethylhexylsalicylat, Ethylhexyltriazon, Octocrylen, Homosalat, Polysilikon-15 oder Mischungen davon umfasst.

9. Stabile Körperpflegezusammensetzung nach einem der Ansprüche 1 bis 7, wobei das polare Öl Butylphthalimid, Isopropylphthalimid, Phenylethylbenzoat, Dicaprylylcarbonat, Isopropyllauroylsarcosinat, Butyloctylsalicylat, Dioctylmalat, Dicaprylylmaleat, Diisopropyladipat, Isononylisononanoat, Isopropylisostearat, Propylenglycoldicaprat, C12-15-Alkoholbenzoat, Derivate oder Mischungen davon umfasst.

10. Stabile Körperpflegezusammensetzung nach einem der Ansprüche 1 bis 7, wobei das polare Öl Folgendes umfasst:
(i) ein Sonnenschutzmittel, wobei das Sonnenschutzmittel Benzophenon-3, Bisethylhexyloxyphenolmethoxyphenyltriazin, Butylmethoxydibenzoylmethan, Diethylaminohydroxybenzoylhexylbenzoat, Ethylhexylmethoxycinnamat, Ethylhexylsalicylat, Ethylhexyltriazon, Octocrylen, Homosalat, Polysilikon-15 oder Mischungen davon umfasst; und
(ii) ein Element, das Butylphthalimid, Isopropylphthalimid, Phenylethylbenzoat, Dicaprylylcarbonat, Isopropyllauroylsarcosinat, Butyloctylsalicylat, Dioctylmalat, Dicaprylylmaleat, Diisopropyladipat, Isononylisononanoat, Isopropylisostearat, Propylenglycoldicaprat, C12-15-Alkoholbenzoat, Derivate oder Mischungen davon umfasst.

11. Stabile Körperpflegezusammensetzung nach einem der vorstehenden Ansprüche, die ferner einen Hautpflegewirkstoff umfasst, wobei der Hautpflegewirkstoff Vitamine, Peptide, Zuckeramine, Ölregulierungsmittel, Bräunungsmittel, aknehemmende Wirkstoffe, Abschuppungswirkstoffe, Anticellulitiswirkstoffe, Komplexbildner, Hautaufheller, Flavonoide, Proteaseinhibitoren, vitaminfreie Antioxidationsmittel und Radikalfänger, Haarwuchsregulatoren, Antifaltenwirkstoffe, Antiatrophiewirkstoffe, Mineralien, Phytosterole und/oder Pflanzenhormone, Tyrosinaseinhibitoren, entzündungshemmende Mittel, N-Acylaminosäure-Verbindungen, antimikrobielle Mittel, pilzbefallverhütende Mittel oder Mischungen davon umfasst.

12. Stabile Körperpflegezusammensetzung nach einem der vorstehenden Ansprüche, wobei die Zusammensetzung von 10 Gew.-% bis 50 Gew.-% eine Wasserphase umfasst.

## Revendications

1. Composition de soin personnel stable sous la forme d'une émulsion eau-dans-huile comprenant :
a) de 0,01 % en poids à 30 % en poids d'un élastomère à base de silicone comprenant un groupe polyalkyl éther de Formule I ou II : 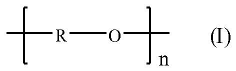 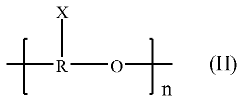 dans laquelle R est un groupe alkyle comportant 3 atomes de carbone ou plus dans une orientation linéaire ou ramifiée, de préférence dans laquelle R est isopropyle ou n-propyle et dans laquelle X est un substituant amine, ester, amide, cétone, aldéhyde, nitro, éther, énol, carboxyle, carbonyle, halogénure, benzyle, ou aromatique, et dans laquelle n est un nombre entier compris entre 2 et 30 ;
b) de 0 % en poids à 20 % en poids d'un élastomère à base de silicone non émulsifiant ;
c) de 0,05 % en poids à 20 % en poids d'un émulsifiant ;
d) plus de 10 % en poids d'une huile polaire ; et
e) une phase aqueuse comprenant de l'eau.

2. Composition de soin personnel stable selon la revendication 1, dans laquelle l'élastomère à base de silicone comprenant le groupe polyalkyl éther comprend un squelette de polysiloxane et une chaîne greffée s'étendant à partir du polysiloxane, dans laquelle la chaîne greffée comprend le groupe polyalkyl éther.

3. Composition de soin personnel stable selon la revendication 1, dans laquelle l'élastomère à base de silicone comprenant le groupe polyalkyl éther comprend au moins deux squelettes de polysiloxane reliés par une chaîne de réticulation, dans laquelle la chaîne de réticulation comprend le groupe polyalkyl éther.

4. Composition de soin personnel stable selon la revendication 1, dans laquelle l'élastomère à base de silicone comprenant le groupe polyalkyl éther est choisi parmi un groupe constitué de polymère réticulé bis-vinyldiméthicone/bis-isobutyl PPG-20, polymère réticulé bis-vinyldiméthicone/PPG-20, polymère réticulé diméthicone/bis-isobutyl PPG-20, polymère réticulé diméthicone/PPG-20, et polymère réticulé diméthicone/bis-secbutyl PPG-20.

5. Composition de soin personnel stable selon l'une quelconque des revendications précédentes, dans laquelle l'élastomère à base de silicone non émulsifiant comprend des polymères réticulés de diméthicone, des polymères réticulés diméthicone/vinyl diméthicone, des polymères réticulés alkyl en C30-45 cétéaryl diméthicone, des polymères réticulés cétéaryl diméthicone, des polymères réticulés diméthicone/phényl vinyl diméthicone, des polymères réticulés vinyl diméthicone/lauryl diméthicone, des polymères réticulés trifluoropropyl diméthicone/trifluoropropyl divinyldiméthicone, et leurs mélanges.

6. Composition de soin personnel stable selon l'une quelconque des revendications précédentes, dans laquelle l'émulsifiant est un émulsifiant à base de silicone réticulé dépourvu de groupe polyalkyl éther de Formule I ou II, dans laquelle R est un groupe alkyle comportant 3 atomes de carbone ou plus dans une orientation linéaire ou ramifiée et X est un substituant amine, ester, amide, cétone, aldéhyde, nitro, éther, énol, carboxyle, carbonyle, halogénure, benzyle, ou aromatique, et n est un nombre entier compris entre 2 et 30 ; de préférence ledit émulsifiant est un élastomère à base de silicone réticulé à fonction polyoxyalkylène ou polyglycérol.

7. Composition de soin personnel stable selon l'une quelconque des revendications 1 à 6, dans laquelle l'émulsifiant est d'une structure linéaire ou ramifiée comprenant des silicones à modification polyéther, des silicones à modification polyglycérine, et des silicones à modification conjointe polyéther / alkyle, ou des silicones à modification conjointe polyglycérine / alkyle.

8. Composition de soin personnel stable selon l'une quelconque des revendications précédentes, dans laquelle l'huile polaire comprend un écran solaire, de préférence l'écran solaire comprend du benzophénone-3, du bis-éthylhexyloxyphénol de la méthoxyphényl triazine, du butyl méthoxydibenzoyl-méthane, du benzoate de diéthylamino hydroxy-benzoyl hexyle, du méthoxy-cinnamate d'éthylhexyle, du salicylate d'éthylhexyle, de l'éthylhexyl triazone, de l'octocrylène, de l'homosalate, de la polysilicone-15, et leurs mélanges.

9. Composition de soin personnel stable selon l'une quelconque des revendications 1 à 7, dans laquelle l'huile polaire comprend du butyl phtalimide, de l'isopropyl phtalimide, du benzoate de phényléthyle, du carbonate de dicaprylyle, du lauroylsarcosinate d'isopropyle, de l'octylsalicylate de butyle, du malate de dioctyle, du maléate de dicaprylyle, de l'adipate de di-isopropyle, de l'isononanoate d'isononyle, de l'isostéarate d'isopropyle, du dicaprate de propylène glycol, du benzoate d'alcool en C12 à 15, leurs dérivés et leurs mélanges.

10. Composition de soin personnel stable selon l'une quelconque des revendications 1 à 7, dans laquelle l'huile polaire comprend :
(i) un écran solaire, l'écran solaire comprenant du benzophénone-3, du bis-éthylhexyloxyphénol, de la méthoxyphényl triazine, du butyl méthoxydibenzoyl-méthane, du benzoate de diéthylamino hydroxy-benzoyl hexyle, du méthoxy-cinnamate d'éthylhexyle, du salicylate d'éthylhexyle, de l'éthylhexyl triazone, de l'octocrylène, de l'homosalate, de la polysilicone-15, et leurs mélanges ; et
(ii) un élément comprenant du butyl phtalimide, de l'isopropyl phtalimide, du benzoate de phényléthyle, du carbonate de dicaprylyle, du lauroylsarcosinate d'isopropyle, de l'octylsalicylate de butyle, du malate de dioctyle, du maléate de dicaprylyle, de l'adipate de di-isopropyle, de l'isononanoate d'isononyle, de l'isostéarate d'isopropyle, du dicaprate de propylène glycol, du benzoate d'alcool en C12 à 15, leurs dérivés et leurs mélanges.

11. Composition de soin personnel stable selon l'une quelconque des revendications précédentes, comprenant en outre un principe actif pour le soin de la peau, le principe actif pour le soin de la peau comprenant des vitamines, des peptides, des amines de sucre, des agents de contrôle d'huile, des principes actifs de bronzage, des principes actifs anti-acnéiques, des principes actifs de desquamation, des principes actifs anti-cellulite, des agents chélatants, des agents de décoloration de la peau, des flavonoïdes, des inhibiteurs de protéase, des antioxydants non-vitamines et des agents anti-radicaux libres, des régulateurs de la pousse des cheveux, des principes actifs anti-rides, des principes actifs anti-atrophie, des minéraux, des phytostérols et/ou hormones végétales, des inhibiteurs de tyrosinase, des agents anti-inflammatoires, des composés à base d'acide N-acyl aminé, des antimicrobiens, des antifongiques, et leurs mélanges.

12. Composition de soin personnel stable selon l'une quelconque des revendications précédentes, où la composition comprend de 10 % en poids à 50 % en poids de phase aqueuse.
